# EUROPEAN PATENT SPECIFICATION

(11) **EP 2 394 162 B1**
(45) Date of publication and mention of the grant of the patent: **01.05.2013**
(21) Application number: 10722730.8
(22) Date of filing: 11.03.2010
(51) Int. Cl.: G01N 33/36, G01N 21/25, G01N 21/898

(54) **MEASUREMENT OF TEXTILE FABRICS**
MESSUNG VON TEXTILGEWEBEN
MESURE DE TISSUS TEXTILES

(30) Priority: 20.03.2009 GB 0904833
(43) Date of publication of application: 14.12.2011
(73) Proprietor: Nixtex Limited, Nottingham NG 1 6AS (GB)
(72) Inventor: NIXON, Christopher, Nottingham NG1 6AS (GB); THORNE, Andrew, Nottingham NG 1 6AS (GB)
(74) Representative: Swindell & Pearson Limited
(86) International application number: PCT/GB2010/000442
(87) International publication number: WO 2010/106306

(56) References cited:
- WO-A1-92/08967
- WO-A1-97/29368
- DE-A1- 3 639 636
- GB-A- 2 272 516
- JP-A- 8 015 030

## Description

The present invention relates to measurement of textile fabrics. In particular, the invention relates to measurement of fabric colour, shade or the like.

German patent application no. DE 3639636 (Massen) discloses a method and a circuit arrangement for automatic inspection of plane fabric webs by means of a parallel arrangement of colour-area cameras. The inspection is based on colour-defect recognition carried out in real time, at the same time as local structural-defect recognition carried out in real time, and on two-dimensional image evaluation initiated in the event of unreliable recognition results and no longer carried out in real time.

Example embodiments of the present invention provide apparatus comprising:
a sensor having an associated sensor field;
a feed arrangement operable to present an area of a textile fabric length at the sensor field;
the feed arrangement being further operable to provide relative movement between the sensor and the textile fabric length to present a sequence of areas of fabric at the sensor field;
the sensor being repeatedly operable to provide a colour output representing fabric colour and measured from respective areas of the fabric sequentially presented at the sensor field;
and the feed arrangement being operable, each time the sensor provides a colour output, to provide a respective position output representing the position of the sensor relative to the fabric,
the feed arrangement and sensor thereby providing a sequence of colour outputs measured from known areas of the fabric represented by the respective position outputs.

In this specification, the term "colour" is used to refer to hue, shade, reflectivity or any other parameter of visual appearance which is visible to the eye.

The feed arrangement may be operable to provide relative movement by moving the textile fabric length. The feed arrangement may be operable to unroll the textile fabric length from a roll and to present an unrolled area at the sensor field. The textile fabric length may be unrolled from a first roll and rolled to a second roll, the fabric being presented at the sensor field while unrolled between the first roll and the second roll. The sequence of areas presented at the sensor field may form a line of positions along the textile fabric length.

There may be a plurality of sensors as aforesaid, each having a respective associated sensor field. The plurality of sensors may be arrayed to measure from respective lines of fabric areas, as the textile fabric length moves relative to the sensors. The sensor fields of the plurality of sensors may be arrayed substantially perpendicular to the direction of relative movement of the textile fabric length and the sensors.

The apparatus may further comprise memory means operable to store the colour outputs and respective position outputs. The colour outputs and respective position outputs may be collated to form a dataset representing a map of the textile fabric length. The apparatus may further comprise a visual display means, the visual display means being operable to provide a visual representation of the map.

The sensor may provide colour outputs in RGB format. The apparatus may further comprise control means operable to analyse the colour outputs. The control means may be operable to analyse the colour outputs from each fabric length to assign an overall colour measurement for each fabric length. The control means may be operable to determine the range of colours represented by the colour outputs for a fabric length. The control means may be operable to determine if the range of colours is greater than a permitted maximum range. The control means may be operable to compare colour outputs to identify matching areas of the fabric by reference to the position outputs. The control means may be operable to analyse the colour outputs in order to calculate Delta E values.

Examples of the invention also provide a method comprising:
receiving a sequence of colour outputs representing the fabric colour of a sequence of areas of a textile fabric length;
receiving a respective position output for each of the colour outputs;
and determining the position of each of the sequence of areas on the textile fabric length;
thereby providing a sequence of colour outputs measured from known areas of the fabric represented by the respective position outputs.

A feed arrangement may be used to provide relative movement between a sensor and the textile fabric length to present a sequence of areas of fabric at the sensor.

Relative movement may be provided by moving the textile fabric length. The textile fabric length may be unrolled from a roll, an unrolled area being presented at the sensor. The textile fabric length may be unrolled from a first roll and rolled to a second roll, the fabric being presented at the sensor while unrolled between the first roll and the second roll. The sequence of areas presented at the sensor may form a line of positions along the textile fabric length.

There may be a plurality of sensors. The plurality of sensors may be arrayed to measure from respective lines of fabric areas, as the textile fabric length moves relative to the sensors. The sensor fields of the plurality of sensors may be arrayed substantially perpendicular to the direction of relative movement of the textile fabric length and the sensors.

The colour outputs and respective position outputs may be stored. The colour outputs and respective position outputs may be collated to form a dataset representing a map of the textile fabric length. A visual display means may be used to provide a visual representation of the map.

The colour outputs may be analysed. The colour outputs from each fabric length are analysed to assign an overall colour measurement for each fabric length. The colour outputs may be analysed to determine the range of colours represented by the colour outputs for a fabric length. The method may determine if the range of colours is greater than a permitted maximum range. The method may compare colour outputs to identify matching areas of the fabric by reference to the position outputs. The method may analyse the colour outputs in order to calculate Delta E values.

The invention also provides software which, when installed on a computer system, is operable to perform the whole or any part of the preceding method. The invention also provides a computer readable medium having machine-readable instructions recorded thereon and representing software as aforesaid.

In another aspect, examples of the invention provide a batch of fabric panels cut from fabric lengths, the fabric panels being matched in accordance with colour outputs and position outputs obtained by means of the apparatus set out above, or in accordance with the method set out above.

Examples of the present invention will now be described in more detail, by way of example only, and with reference to the accompanying drawings, in which:

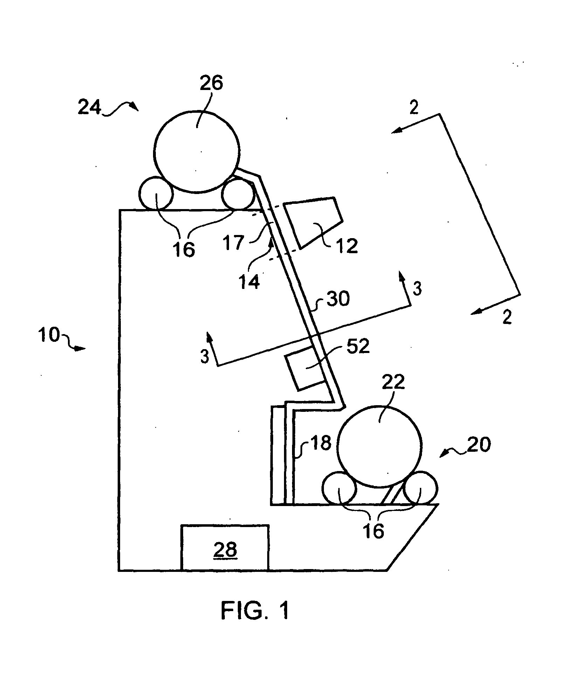

Fig. 1 is a schematic side view of an example apparatus for use in implementing the invention;
Figs. 2 and 3 are schematic views of the apparatus of Fig. 1; at the lines 2-2 and 3-3 in Fig. 1;
Fig. 4 is an enlarged schematic view of a sensor;
Fig. 5 illustrates a display of the device; and
Fig. 6 illustrates an example method of the invention.

The drawings illustrate apparatus 10 comprising at least one sensor 12 having an associated sensor field indicated generally at 14. A feed arrangement 16 is operable to present an area 17 of a textile fabric length 18 at the sensor field 14. The feed arrangement 16 also provides relative movement between the sensor 12 and the textile fabric length 18 to present a sequence of areas 17 of fabric at the sensor field 14. The sensor 12 is repeatedly operated, as will be described, to provide a colour output representing fabric colour and measured from respective areas 17 of the fabric, as sequentially presented at the sensor field 14. The feed arrangement 16 operates, each time the sensor 12 provides a colour output, to provide a respective position output representing the position of the sensor 12 relative to the fabric 18. The feed arrangement 16 and sensor 12 thereby providing a sequence of colour outputs measured from known areas 17 of the fabric 18, represented by the respective position outputs.

In more detail, the apparatus 10 has an unrolling station 20 for receiving a roll 22 of textile fabric. A rolling station 24 is provided for receiving an initially empty roll 26. The apparatus 10 has a drive mechanism illustrated schematically at 28 and defines a path 30 for a web of fabric 18 to pass from the roll 22, along the path 30, to the empty roll 26. Accordingly, the feed arrangement 16, including the drive mechanism 28, provides relative movement between the textile fabric length 18 and the sensor 12, by moving the textile fabric length 18. Alternatively, the sensor 12 could be moved to scan the surface of the length 18, but the extreme length of fabric rolls used commercially (which may be up to 5 km in length) makes it more practical to move the fabric past the sensor 12. A combination of movement of the sensor 12 and the fabric length 18 could be used. As the textile fabric length moves along the path 30 from the roll 22 to the roll 26, a line of positions along the textile fabric length 18 will be sequentially presented to the sensor 12.

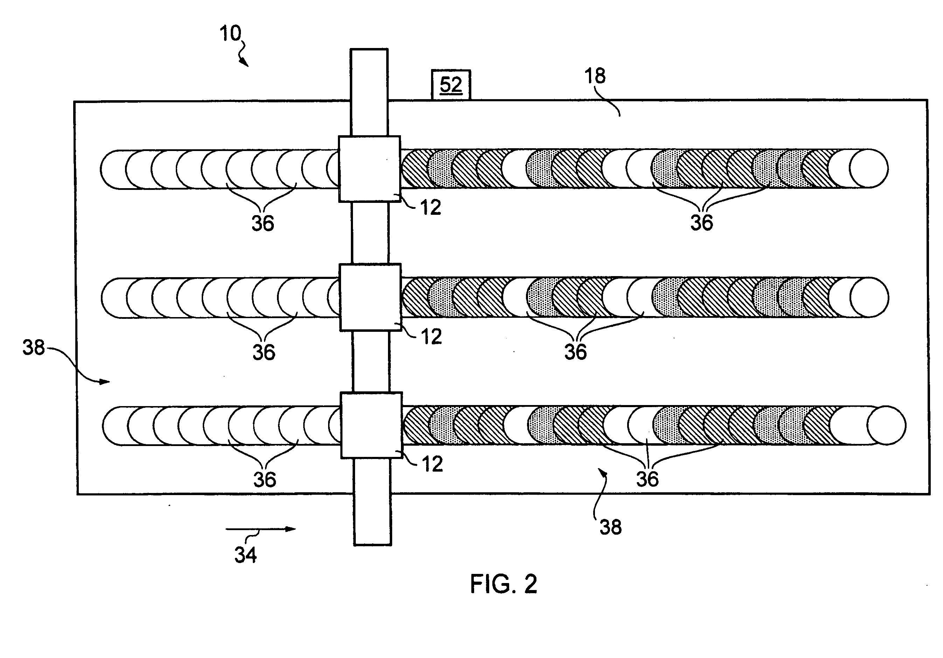

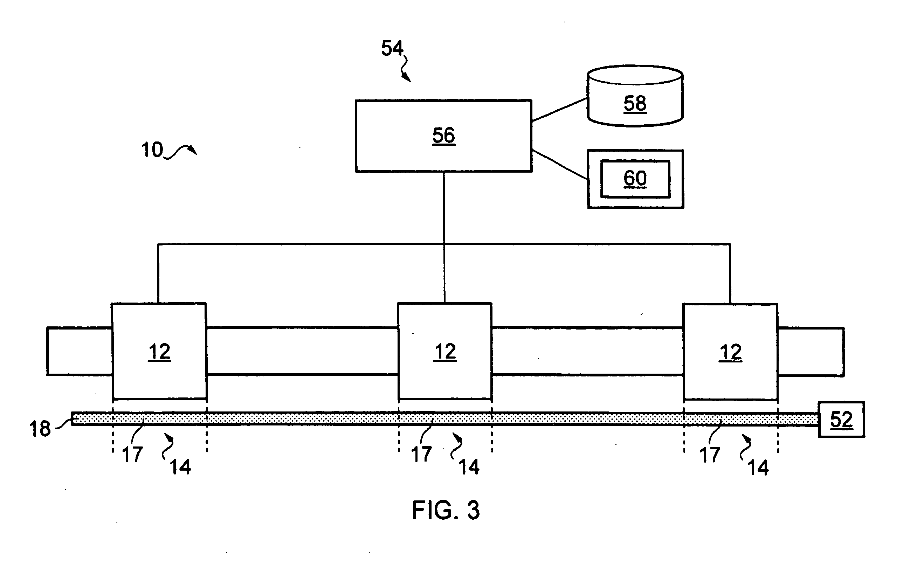

Figures 2 and 3 illustrate the textile fabric length 18 as it moves along the path 30 of the apparatus 10. The fabric length 18 moves past the sensor 12 which, in this example, includes three sensor heads 12. A different number of sensor heads 12 could be used. It is envisaged that as many as thirty sensor heads 12 could be used together. Each sensor head 12 has a respective sensor field 14 associated with it, as will be described. The sensor heads 12 are arrayed, in this example, in a line which is generally perpendicular with the direction of movement of the textile fabric length 18 relative to the sensor heads 12, this direction being indicated in Figure 2 by an arrow 34. As the fabric length 18 moves past the sensor heads 12, a line of positions 36 passes each sensor head 12. The positions 36 are diagrammatically illustrated in Figure 2 as overlapping circular regions. In the region 38, before the fabric 18 reaches the sensor heads 12, each position 36 is plain, for reasons which will become apparent. In the region 40, after the fabric 18 has passed the sensor head 12, each position 36 is shaded, for reasons which will become apparent.

It can be seen from Figure 2 that the sensor heads 12 define respective lines of positions 36 which run parallel along the length of the fabric length 18. As the textile fabric length 18 is fully unrolled from the roll 22 to the empty roll 26, the lines of positions 36 will extend along the whole length of the fabric length 18.

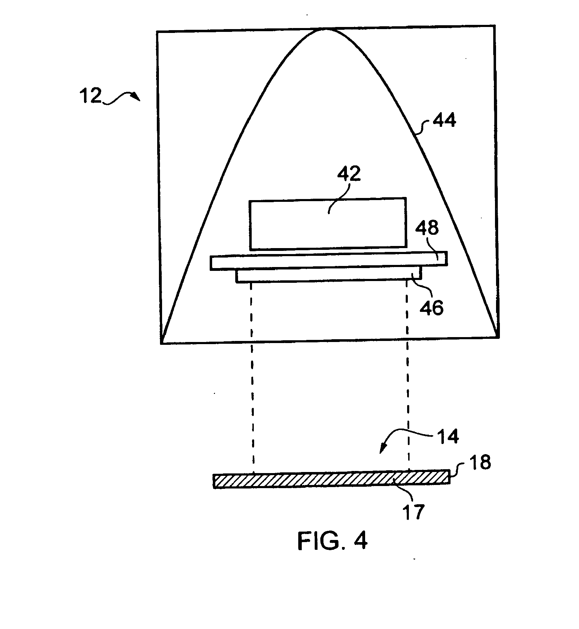

One of the sensor heads 12 is illustrated in more detail in Figure 4. The sensor head 12 includes a transducer 42 housed within a shroud 44 to reduce the effects of ambient light, reflections etc. A light source 46 is provided to create reliable, consistent lighting in the vicinity of the head 12. Control electronics 48 or other apparatus associated with the head 12 may also be housed within the shroud 44, which may act as a reflector to cast the output of the light source 46 onto the textile fabric length 18. The transducer 42 has a viewing angle 50, illustrated by broken lines in Figure 4, which therefore defines a sensor field 14 at the fabric length 18. As the textile fabric length 18 moves past the sensor head 12, a sequence of areas of fabric will be presented to the sensor head 12.

The transducer 42 of each sensor head 12 is able to take a measurement from the corresponding sensor field 14, representing the fabric colour seen by the transducer 42 at the field 14. Suitable transducers for measuring colour are known in themselves and may report in a number of standard formats. One format is known as RGB format, and is used in this example. RGB format allows other formats to be calculated from it, if required or desired.

The fabric colour is therefore measured without damaging the fabric.

The apparatus 10 also includes a position encoder 52 which detects the movement of the textile fabric length 18 along the path 30. Accordingly, the encoder 52 can report the length of fabric which has passed. This position, together with the relative positions of the encoder 52 and the sensor heads 12 allows the determination of the current position of each of the sensor fields 14 on the textile fabric length 18. That is, each time a sensor head 12 provides a colour output representing fabric colour, measured from the respective sensor field 14, a position output can be taken from the position encoder 52. This links the colour output to a particular position on the fabric length 18, as has just been explained.

It is for this reason that the regions 38, prior to the sensor 12, are plain, because no information is yet known about fabric colour in the region 38. However, the regions 40 have passed the sensor 12, colour outputs have been obtained from the sensor heads 12 and position outputs have been obtained from the position encoder 52, so that the colour of the regions 40, and their position, are both now known and are therefore illustrated in Figure 2 by the simple diagrammatic schema of dark areas, light areas and shaded areas, to indicate three different colours. It should be understood that in a practical example, using conventional colour measurement techniques, many more than three discrete colour states would be identified.

The colour outputs from the sensor heads 12, and the position outputs from the position encoder 52 are all fed to a control arrangement 54 which may, for example, be implemented by means of appropriate software running on a general purpose computer. For the purpose of understanding the present invention, it is sufficient to explain that the control arrangement 54 includes a data processing element 56, associated memory 58 and a visual display unit 60. In this example, all colour outputs and respective position outputs received from the sensor heads 12 and position encoder 52 are stored in the memory 58 of the data-processing element 56. Further data-processing can then take place. In particular, the correspondence between each colour output and its respective position output allows the data to be collated to form a dataset which represents a map of the colour of the textile fabric length 18. This map, as it is being formed, is indicated in Figure 2 by the appearance of the regions 40.

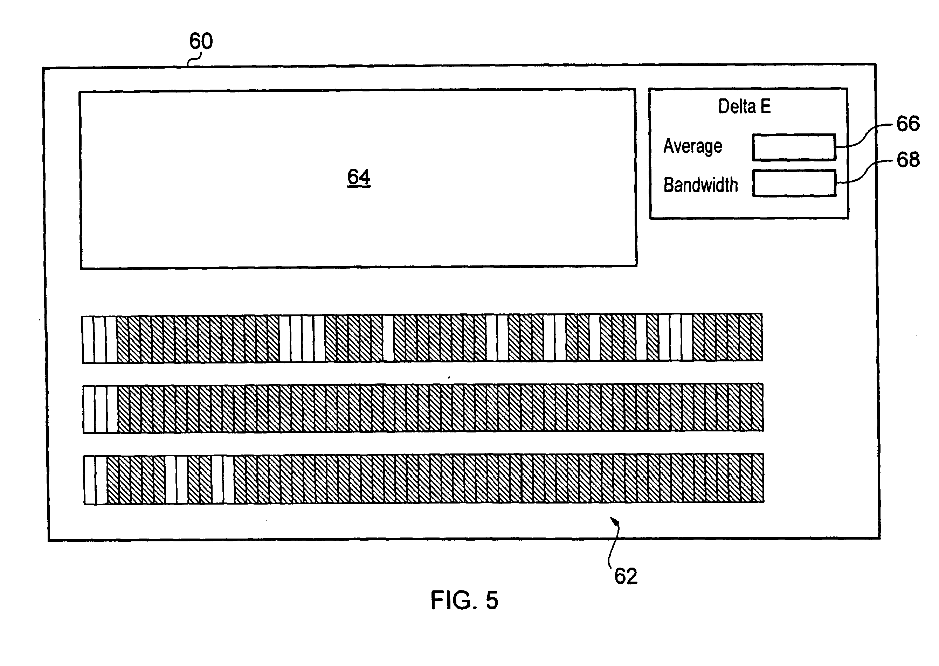

The map may be presented to the user by means of the visual display unit 60, under control from the data-processing element 56. Figure 5 illustrates an example of a display. The lower region 62 shows a map collated from the colour outputs and respective position outputs. The map may show the detected colours, or be in any other appropriate format to provide the required information to the user. For example, the data-processing element 56 may analyse the colour outputs and present the results of the analysis in the region 62, rather than presenting the actual colours detected. The analysis may determine any areas whose colour varies from a target colour by more than a maximum allowable amount. Variation may be measured by a conventional colour measurement technique. In this example, variation is calculated as a Delta E value calculated by the data-processing element 56 from RGB outputs from the sensor heads 12. Delta E values measure the variance of a colour or shade from the intended value. A Delta E value greater than unity indicates a difference which is visible to the human eye. A Delta E value less than unity indicates a difference which is not visible to the human eye.

Alternatively, the analysis may compare colour outputs from various different areas across the fabric length 18 in other ways, to identify those areas which provide an acceptably close visual match (for garment manufacture, for example).

Accordingly, the map shown in the region 62 may indicate areas which match or which lie outside the permitted colour range for the particular roll, rather than showing the actual colour detected.

Additional information can be provided in the upper region 64 of the display 60, such as information identifying the roll of fabric, its manufacturer, intended colour etc. Other fields may include an average Delta E value 66 for the whole of the textile fabric length 18, and an indication 68 of bandwidth (variation) of Delta E values across the length 18.

Analysis of the colour outputs and position outputs may be executed locally, at the apparatus 10, or remotely. In the case of remote analysis, this may take place within the same premises, or the data may be transmitted by any appropriate medium, including the Internet, for analysis elsewhere.

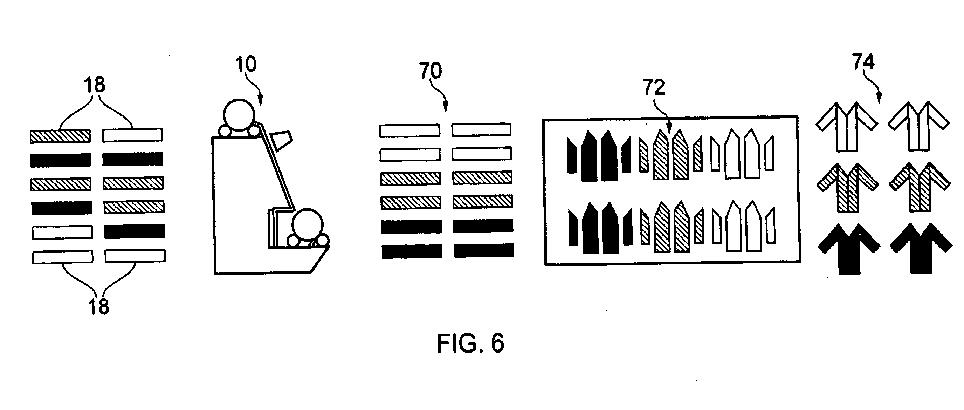

Figure 6 schematically represents a complete process for manufacturing items of clothing from textile fabric, to provide an example of the manner in which examples of the present invention may be used.

In Figure 6, a batch of textile fabric lengths 18 have been received from a manufacturer. These are all intended to have a particular intended colour, but in practice, there will be colour variations between lengths, and within a single length. The lengths 18 are schematically illustrated as bars of block shading or line shading, or unshaded to indicate different colours. Again, only three alternatives are shown, solely for illustrative purposes. In a practical example, the fabric lengths 18 will usually be rolls of fabric.

Each of the textile fabric lengths 18 is first inspected by passing the roll through the apparatus 10, described above. In this first example, this allows a Delta E value to be measured for each length 18, representing the average variance of the colour of that length from the intended colour for the roll. Accordingly, the lengths 18 can be sorted (indicated at 70) to group them into matching colour groups. That is, the average variance of two rolls may indicate that the fabric of those two rolls will match tolerably for garment manufacture, even though those rolls will not acceptably match with other rolls of the batch. The rolls are allocated to groups on this basis. Each length 18 can then be cut into panels 72 from which garments can be made. The resulting panels 72 indicated in Figure 6 are kept in groups corresponding with the groups of rolls from which they were cut and thus, they remain grouped according to their colour. This grouping readily allows garments 74 to be constructed from groups of matching panels 72, so that the resulting garments 74 will pass quality control checks in relation to colour match, even though there will be variation between garments made from different groups. Thus, it is expected that by grouping panels 72 only from lengths 18 which match each other, to create groups of panels 72 which match each other, the risk of creating a single garment 74 from several panels 72 which do not match each other can be significantly reduced, so that the rejection rate of finished garments 74 can also be significantly reduced.

In the example just described, many colour measurements made across and along each roll are used to assign an overall colour measurement to that roll. It is the overall measurement which then determines the grouping of rolls, and hence the grouping of garment panels.

In a further example, the apparatus 10 is used to form a map of each length 18, and the lengths 18 are then cut into panels 72 with due regard to the colours indicated by the maps. Thus, a length 18 which has colour variation within it, can be used to contribute panels 72 to different groups. That is, panels cut from within the single length 18 are grouped with other panels of the same colour variation cut from the same length 18 or from another length 18. Again, it is envisaged that by grouping panels 72 according to their colour, and facilitating this grouping by forming the map by operation of the apparatus 10, there will be a significantly reduced risk of garments 74 being formed from panels 72 which do not match each other.

In this second example panels are grouped according to the colour of the panel itself, regardless of colour variation elsewhere on the same roll.

We envisage that in many practical situations, it will be possible to produce garments of adequate quality even if some of the lengths 18 do not match each other, or do not have consistent colour along their length. This will result in garments 74 which vary in colour from garment to garment, but which have acceptable panel matching within each garment. However, in the event that any length 18 is found to be unacceptably variant from the intended colour, as detected by the apparatus 10, the whole length 18 or the detected part of the length 18 can be rejected.

Wastage arising from rejection of finished garments is therefore expected to be significantly reduced.

Many variations and modifications may be made to the apparatus described above. For example, many technologies and techniques could be used for the sensors and for processing, using and displaying the data.

## Claims

1. Apparatus (10) comprising:
a sensor (12) having an associated sensor field (14);
a feed arrangement (16) operable to present an area (17) of a textile fabric length (18) at the sensor field;
the feed arrangement being further operable to provide relative movement between the sensor and the textile fabric length to present a sequence of areas of fabric at the sensor field;
the sensor being repeatedly operable to provide a colour output representing fabric colour and measured from respective areas of the fabric sequentially presented at the sensor field;
and the feed arrangement being operable, each time the sensor provides a colour output, to provide a respective position output representing the position of the sensor relative to the fabric,
the feed arrangement and sensor thereby providing a sequence of colour outputs measured from known areas of the fabric represented by the respective position outputs.

2. Apparatus (10) according to claim 1, wherein the feed arrangement (16) is operable to provide relative movement by moving the textile fabric length (18).

3. Apparatus (10) according to claim 1 or 2, wherein the feed arrangement (16) is operable to unroll the textile fabric length (18) from a roll (22) and to present an unrolled area at the sensor field (14).

4. Apparatus (10) according to any preceding claim, wherein the textile fabric length (18) is unrolled from a first roll (22) and rolled to a second roll (26), the fabric being presented at the sensor field (14) while unrolled between the first roll and the second roll.

5. Apparatus (10) according to any preceding claim, wherein the sequence of areas presented at the sensor field (14) form a line of positions (36) along the textile fabric length (18).

6. Apparatus (10) according to any preceding claim, wherein there is a plurality of sensors (12) as aforesaid, each having a respective associated sensor field (14).

7. Apparatus (10) according to claim 6, wherein the plurality of sensors (12) is arrayed to measure from respective lines (36) of fabric areas, as the textile fabric length (18) moves relative to the sensors.

8. Apparatus (10) according to claim 6 or 7, wherein the sensor fields (14) of the plurality of sensors (12) is arrayed substantially perpendicular to the direction of relative movement of the textile fabric length (18) and the sensors.

9. Apparatus (10) according to any preceding claim, wherein the apparatus further comprises memory means (58) operable to store the colour outputs and respective position outputs.

10. Apparatus (10) according to any preceding claim, wherein the colour outputs and respective position outputs are collated to form a dataset representing a map (62) of the textile fabric length (18).

11. Apparatus (10) according to claim 10, wherein the apparatus further comprises a visual display means (60), the visual display means being operable to provide a visual representation of the map (62).

12. Apparatus (10) according to any preceding claim, wherein the sensor (12) provides colour outputs in RGB format.

13. Apparatus (10) according to any preceding claim, wherein the apparatus further comprises control means (54) operable to analyse the colour outputs.

14. Apparatus (10) according to claim 13, wherein the control means (54) is operable to analyse the colour outputs from each fabric length (18) to assign an overall colour measurement for each fabric length.

15. Apparatus (10) according to claim 13 or 14, wherein the control means (54) is operable to determine the range of colours represented by the colour outputs for a fabric length (18).

16. Apparatus (10) according to claim 15, wherein the control means (54) is operable to determine if the range of colours is greater than a permitted maximum range.

17. Apparatus (10) according to any of claims 13 to 16, wherein the control means (54) is operable to compare colour outputs to identify matching areas of the fabric (18) by reference to the position outputs.

18. Apparatus (10) according to any of claims 13 to 17, wherein the control means (54) is operable to analyse the colour outputs in order to calculate Delta E values.

19. A method comprising:
receiving a sequence of colour outputs representing the fabric colour of a sequence of areas of a textile fabric length (18);
receiving a respective position output for each of the colour outputs;
and determining the position of each of the sequence of areas on the textile fabric length;
thereby providing a sequence of colour outputs measured from known areas of the fabric represented by the respective position outputs.

20. A method according to claim 19, wherein a feed arrangement (16) is used to provide relative movement between a sensor (12) and the textile fabric length (18) to present a sequence of areas of fabric at the sensor.

21. A method according to claim 20, wherein relative movement is provided by moving the textile fabric length (18).

22. A method according to claims 20 or 21, wherein the textile fabric length (18) is unrolled from a roll (22), an unrolled area being presented at the sensor (12).

23. A method according to any of claims 20 to 22, wherein the textile fabric length (18) is unrolled from a first roll (22) and rolled to a second roll (26), the fabric being presented at the sensor (12) while unrolled between the first roll and the second roll.

24. A method according to any of claims 20 to 23, wherein the sequence of areas presented at the sensor (12) forms a line of positions (36) along the textile fabric length (18).

25. A method according to any of claims 20 to 24, wherein there is a plurality of sensors (12).

26. A method according to claim 25, wherein the plurality of sensors (12) is arrayed to measure from respective lines (36) of fabric areas, as the textile fabric length (18) moves relative to the sensors.

27. A method according to claims 25 or 26, wherein the sensor fields (14) of the plurality of sensors (12) are arrayed substantially perpendicular to the direction of relative movement of the textile fabric length (18) and the sensors.

28. A method according to any of claims 19 to 27, wherein the colour outputs and respective position outputs are stored.

29. A method according to any of claims 19 to 28, wherein the colour outputs and respective position outputs are collated to form a dataset representing a map (62) of the textile fabric length (18).

30. A method according to claim 29, wherein a visual display means (60) is used to provide a visual representation of the map (62).

31. A method according to any of claims 19 to 30, wherein the colour outputs are analysed.

32. A method according to any of claims 19 to 31, wherein the colour outputs from each fabric length (18) are analysed to assign an overall colour measurement for each fabric length.

33. A method according to any of claims 19 to 32, wherein the colour outputs are analysed to determine the range of colours represented by the colour outputs for a fabric length (18).

34. A method according to claim 33, wherein the method determines if the range of colours is greater than a permitted maximum range.

35. A method according to any of claims 19 to 34, wherein the method compares colour outputs to identify matching areas of the fabric (18) by reference to the position outputs.

36. A method according to any of claims 19 to 35, wherein the method analyses the colour outputs in order to calculate Delta E values.

37. Software which, when installed on a computer system (60), is operable to perform the method of any of claim 19 to 36.

38. A computer readable medium having machine-readable instructions recorded thereon and representing software according to claim 37.

39. A batch of fabric panels (72) cut from fabric lengths (18), the fabric panels being matched in accordance with colour outputs and position outputs obtained by means of the apparatus (10) of claims 1 to 18.

40. A batch of fabric panels (72) cut from fabric lengths (18), the fabric panels being matched in accordance with colour outputs and position outputs obtained in accordance with the method of claims 19 to 36.

## Patentansprüche

1. Vorrichtung (10), welche aufweist:
einen Sensor (12) mit einem zugeordneten Sensorfeld (14);
eine Vorschubanordnung (16), die betätigbar ist, um eine Fläche (17) einer gewissen Länge eines Textilgewebes (18) bei dem Sensorfeld darzubieten;
wobei die Vorschubanordnung außerdem betätigbar ist, um eine Relativbewegung zwischen dem Sensor und der gewissen Länge des Textilgewebes zu erzeugen, um eine Sequenz von Gewebeflächen bei dem Sensorfeld darzubieten;
wobei der Sensor wiederholt betätigbar ist, um eine Farbausgabe zu erzeugen, welche die Textilfarbe repräsentiert und von jeweiligen Flächen des Gewebes gemessen wird, welches bei dem Sensorfeld sequentiell dargeboten wird; und wobei die Vorschubanordnung jedesmal, wenn der Sensor eine Farbausgabe erzeugt, betätigbar ist, um eine jeweilige Positionsausgabe zu erzeugen, welche die Position des Sensors relativ zu dem Gewebe repräsentiert;
wobei die Vorschubanordnung und der Sensor dadurch eine Sequenz von Farbausgaben erzeugen; welche von bekannten Flächen des Gewebes gemessen werden, welche durch die jeweiligen Positionsausgaben repräsentiert werden.

2. Vorrichtung (10) nach Anspruch 1, bei welcher die Vorschubanordnung (16) betätigbar ist, um durch Bewegen der gewissen Länge des Textilgewebes (18) eine Relativbewegung zu erzeugen.

3. Vorrichtung (10) nach Anspruch 1 oder 2, bei welcher die Vorschubanordnung (16) betätigbar ist, um die gewisse Länge des Textilgewebes (18) von einer Rolle (22) abzurollen und eine abgerollte Fläche bei dem Sensorfeld (14) darzubieten.

4. Vorrichtung (10) nach einem der vorhergehenden Ansprüche, bei welcher die gewisse Länge des Textilgewebes (18) von einer ersten Rolle (22) abgerollt wird und auf eine zweite Rolle (26) aufgerollt wird, wobei das Gewebe bei dem Sensorfeld (14) dargeboten wird, während es zwischen der ersten Rolle und der zweiten Rolle abgerollt ist.

5. Vorrichtung (10) nach einem der vorhergehenden Ansprüche, bei welcher die Sequenz der bei dem Sensorfeld (14) dargebotenen Flächen eine Linie von Positionen (36) entlang der gewissen Länge des Textilgewebes bilden.

6. Vorrichtung (10) nach einem der vorhergehenden Ansprüche, bei welcher eine Vielzahl der zuvor genannten Sensoren (12) vorhanden ist, wovon jeder ein jeweiliges zugeordnetes Sensorfeld (14) hat.

7. Vorrichtung (10) nach Anspruch 6, bei welcher die Vielzahl der Sensoren (12) als Array angeordnet sind, um von jeweiligen Linien (36) aus Gewebeflächen zu messen, wenn sich die gewisse Länge des Textilgewebes (18) relativ zu den Sensoren bewegt.

8. Vorrichtung (10) nach Anspruch 6 oder 7, bei welcher die Sensorfelder (14) der Vielzahl von Sensoren (12) als Array im wesentlichen senkrecht zur Richtung der Relativbewegung der gewissen Länge des Textilgewebes (18) und der Sensoren angeordnet sind.

9. Vorrichtung (10) nach einem der vorhergehenden Ansprüche, bei welcher die Vorrichtung außerdem Speichermittel (58) aufweist, welche betätigbar sind, um die Farbausgaben und jeweiligen Positionsausgaben zu speichern.

10. Vorrichtung (10) nach einem der vorhergehenden Ansprüche, bei welcher die Farbausgaben und jeweiligen Positionsausgaben kollationiert werden, um einen Datensatz zu bilden, welcher ein Abbild (62) der gewissen Länge des Textilgewebes (18) repräsentiert.

11. Vorrichtung (10) nach Anspruch 10, bei welcher die Vorrichtung eine visuelles Anzeigemittel (60) aufweist, welches betätigbar ist, um eine visuelle Repräsentation des Abbilds (62) zu erzeugen.

12. Vorrichtung (10) nach einem der vorhergehenden Ansprüche, bei welcher der Sensor (12) Farbausgaben im RGB-Format erzeugt.

13. Vorrichtung (10) nach einem der vorhergehenden Ansprüche, bei welcher die Vorrichtung außerdem ein Steuerungsmittel (54) aufweist, welches betätigbar ist, um die Farbausgaben zu analysieren.

14. Vorrichtung (10) nach Anspruch 13, bei welcher das Steuerungsmittel (54) betätigbar ist, um die Farbausgaben von jeder gewissen Länge des Textilgewebes (18) zu analysieren, um für jede gewisse Länge des Textilgewebes (18) eine Gesamt-Farbmessung zuzuordnen.

15. Vorrichtung (10) nach Anspruch 13 oder 14, bei welcher das Steuerungsmittel (54) betätigbar ist, um den Bereich an Farben zu bestimmen, welche durch die Farbausgaben für eine gewisse Länge des Textilgewebes (18) repräsentiert sind.

16. Vorrichtung (10) nach Anspruch 15, bei welcher das Steuerungsmittel (54) betätigbar ist, um zu bestimmen, ob der Bereich an Farben grösser als ein zulässiger maximaler Bereich ist.

17. Vorrichtung (10) nach einem der Ansprüche 13 bis 16, bei welcher das Steuerungsmittel (54) betätigbar ist, um Farbausgaben zu vergleichen, um durch Bezugnahme auf die Positionsausgaben übereinstimmende Flächen des Gewebes (18) zu identifizieren.

18. Vorrichtung (10) nach einem der Ansprüche 13 bis 17, bei welcher das Steuerungsmittel (54) betätigbar ist, um die Farbausgaben zu analysieren, um Delta-E-Werte zu berechnen.

19. Verfahren, welches die folgenden Schritte aufweist:
Empfangen einer Sequenz von Farbausgaben, welche die Gewebefarbe einer Sequenz von Flächen einer gewissen Länge eines Textilgewebes (18) repräsentieren;
Empfangen einer jeweiligen Positionsausgabe für jede der Farbausgaben; und
Bestimmen der Position jeder Fläche der Sequenz von Flächen auf der gewissen Länge des Textilgewebes; und dabei
Erzeugen einer Sequenz von Farbausgaben, welche von bekannten Flächen des Gewebes gemessen werden, welche durch die jeweiligen Positionsausgaben repräsentiert werden.

20. Verfahren nach Anspruch 19, bei welchem eine Vorschubanordnung (16) verwendet wird, um eine Relativbewegung zwischen dem Sensor und der gewissen Länge des Textilgewebes zu erzeugen, um eine Sequenz von Gewebeflächen bei dem Sensor darzubieten.

21. Verfahren nach Anspruch 20, bei welchem durch Bewegen der gewissen Länge des Textilgewebes (18) eine Relativbewegung erzeugt wird.

22. Verfahren nach Anspruch 20 oder 21, bei welchem die gewisse Länge des Textilgewebes (18) von einer Rolle (22) abgerollt wird, wobei eine abgerollte Fläche bei dem Sensor (12) dargeboten wird.

23. Verfahren nach einem der Ansprüche 20 bis 22, bei welchem die gewisse Länge des Textilgewebes (18) von einer ersten Rolle (22) abgerollt wird und auf eine zweite Rolle (26) aufgerollt wird, wobei das Gewebe bei dem Sensor (12) dargeboten wird, während es zwischen der ersten Rolle und der zweiten Rolle abgerollt ist.

24. Verfahren nach einem der Ansprüche 20 bis 23, bei welchem die Sequenz der bei dem Sensor (12) dargebotenen Flächen eine Linie von Positionen (36) entlang der gewissen Länge des Textilgewebes bildet.

25. Verfahren nach einem der Ansprüche 20 bis 24, bei welchem eine Vielzahl von Sensoren (12) vorhanden ist.

26. Verfahren nach Anspruch 25, bei welchem die Vielzahl der Sensoren (12) als Array angeordnet sind, um von jeweiligen Linien (36) aus Gewebeflächen zu messen, wenn sich die gewisse Länge des Textilgewebes (18) relativ zu den Sensoren bewegt.

27. Verfahren nach Anspruch 25 oder 26, bei welchem die Sensorfelder (14) der Vielzahl von Sensoren (12) als Array im wesentlichen senkrecht zur Richtung der Relativbewegung der gewissen Länge des Textilgewebes (18) und der Sensoren angeordnet sind.

28. Verfahren nach einem der Ansprüche 19 bis 27, bei welchem die Farbausgaben und jeweiligen Positionsausgaben gespeichert werden.

29. Verfahren nach einem der Ansprüche 19 bis 28, bei welchem die Farbausgaben und jeweiligen Positionsausgaben kollationiert werden, um einen Datensatz zu bilden, welcher ein Abbild (62) der gewissen Länge des Textilgewebes (18) repräsentiert.

30. Verfahren nach Anspruch 29, bei welchem ein visuelles Anzeigemittel (60) verwendet wird, um eine visuelle Repräsentation des Abbilds (62) zu erzeugen.

31. Verfahren nach einem der Ansprüche 19 bis 30, bei welchem die Farbausgaben analysiert werden.

32. Verfahren nach einem der Ansprüche 19 bis 31, bei welchem die Farbausgaben von jeder gewissen Länge des Textilgewebes (18) analysiert werden, um für jede gewisse Länge des Textilgewebes eine Gesamt-Farbmessung zuzuordnen.

33. Verfahren nach einem der Ansprüche 19 bis 32, bei welchem die Farbausgaben analysiert werden, um einen Bereich an Farben zu bestimmen, welche durch die Farbausgaben für eine gewisse Länge des Textilgewebes (18) repräsentiert werden.

34. Verfahren nach Anspruch 33, bei welchem das Verfahren bestimmt, ob der Bereich an Farben grösser als ein zulässiger maximaler Bereich ist.

35. Verfahren nach einem der Ansprüche 19 bis 34, bei welchem das Verfahren Farbausgaben vergleicht, um durch Bezugnahme auf die Positionsausgaben übereinstimmende Flächen des Gewebes (18) zu identifizieren.

36. Verfahren nach einem der Ansprüche 19 bis 35, bei welchem das Verfahren die Farbausgaben analysiert, um Delta-E-Werte zu berechnen.

37. Software, welche, wenn sie auf einem Rechnersystem (60) installiert ist, zur Durchführung des Verfahrens gemäß einem der Ansprüche 19 bis 36 betätigbar ist.

38. Medium, welches durch einen Rechner lesbar ist, darauf abgespeicherte maschinenlesbare Anweisungen besitzt und Software gemäß Anspruch 37 darstellt.

39. Charge aus Textilstücken (72), welche von gewissen Längen eines Textilgewebes (18) geschnitten sind, wobei die Textilstücke in Übereinstimmung mit Farbausgaben und Positionsausgaben aufeinander abgestimmt sind, welche mittels der Vorrichtung (10) gemäß Anspruch 1 bis 18 gewonnen wurden.

40. Charge aus Textilstücken (72), welche von gewissen Längen eines Textilgewebes (18) geschnitten sind, wobei die Textilstücke in Übereinstimmung mit Farbausgaben und Positionsausgaben aufeinander abgestimmt sind, welche mittels des Verfahrens gemäß Anspruch 19 bis 36 gewonnen wurden.

## Revendications

1. Appareil (10) comprenant :
un capteur (12) ayant un champ de détection associé (14) ;
un dispositif d'alimentation (16) pouvant fonctionner pour présenter une zone (17) d'une longueur de tissu textile (18) au niveau du champ de détection ;
le dispositif d'alimentation pouvant en outre être actionné pour produire un mouvement relatif entre le capteur et la longueur de tissu pour présenter une séquence de zones de tissu au niveau du champ de détection ;
le capteur pouvant être actionné de manière répétée pour fournir un signal de sortie de couleur représentant la couleur du tissu et mesuré à partir de zones respectives du tissu présentées de manière séquentielle au niveau du champ de détection ;
et le dispositif d'alimentation pouvant être actionné, chaque fois que le capteur fournit un signal de sortie de couleur, afin de fournir un signal de sortie de position respectif représentant la position du capteur par rapport au tissu,
le dispositif d'alimentation et de capteur produisant ainsi une séquence de signaux de sortie de couleur mesurés à partir de zones connues du tissu représentées par les signaux de sortie de position respectifs.

2. Appareil (10) selon la revendication 1, dans lequel le dispositif d'alimentation (16) peut être actionné pour produire un mouvement relatif par déplacement de la longueur de tissu textile (18).

3. Appareil (10) selon la revendication 1 ou 2, dans lequel le dispositif d'alimentation (16) peut être actionné pour dérouler la longueur de tissu textile (18) à partir d'un rouleau (22) et pour présenter une zone déroulée au niveau du champ de détection (14).

4. Appareil (10) selon l'une quelconque des revendications précédentes, dans lequel la longueur de tissu textile (18) est déroulée à partir d'un premier rouleau (22) et enroulée sur un second rouleau (26), le tissu étant présenté au niveau du champ de détection (14) pendant qu'il est déroulé entre le premier rouleau et le second rouleau.

5. Appareil (10) selon l'une quelconque des revendications précédentes, dans lequel la séquence de zones présentées au niveau du champ de détection (14) forme une ligne de positions (36) le long de la longueur de tissu textile (18).

6. Appareil (10) selon l'une quelconque des revendications précédentes, dans lequel il existe une pluralité de capteurs (12) comme indiqué précédemment, chacun ayant un champ de détection respectif associé (14).

7. Appareil (10) selon la revendication 6, dans lequel la pluralité de capteurs (12) est disposée de manière à prendre des mesures à partir des lignes respectives (36) de zones de tissu, lorsque la longueur de tissu textile (18) se déplace par rapport aux capteurs.

8. Appareil (10) selon la revendication 6 ou 7, dans lequel les champs de détection (14) de la pluralité de capteurs (12) sont disposés sensiblement perpendiculairement par rapport à la direction du mouvement relatif de la longueur de tissu textile (18) et des capteurs.

9. Appareil (10) selon l'une quelconque des revendications précédentes, dans lequel l'appareil comprend en outre des moyens de mémoire (58) pouvant fonctionner pour stocker les signaux de sortie de couleur et les signaux de sortie de position respectifs.

10. Appareil (10) selon l'une quelconque des revendications précédentes, dans lequel les signaux de sortie de couleur et les signaux de sortie de position respectifs sont assemblés pour former un ensemble de données représentant une carte (62) de la longueur de tissu textile (18).

11. Appareil (10) selon la revendication 10, dans lequel l'appareil comprend en outre un moyen d'affichage visuel (60), le moyen d'affichage visuel pouvant être actionné pour produire une représentation visuelle de la carte (62).

12. Appareil (10) selon l'une quelconque des revendications précédentes, dans lequel le capteur (12) fournit des signaux de sortie de couleur au format RGB.

13. Appareil (10) selon l'une quelconque des revendications précédentes, dans lequel l'appareil comprend en outre des moyens de contrôle (54) pouvant être actionnés pour analyser les signaux de sortie de couleur.

14. Appareil (10) selon la revendication 13, dans lequel les moyens de contrôle (54) peuvent être actionnés pour analyser les signaux de sortie de couleur de chaque longueur de tissu (18) pour attribuer une mesure de couleur globale pour chaque longueur de tissu.

15. Appareil (10) selon la revendication 13 ou 14, dans lequel les moyens de contrôle (54) peuvent être actionnés pour déterminer la gamme de couleurs représentée par les signaux de sortie de couleur pour une longueur de tissu (18).

16. Appareil (10) selon la revendication 15, dans lequel les moyens de contrôle (54) peuvent être actionnés pour déterminer si la gamme de couleurs est supérieure à une gamme maximum autorisée.

17. Appareil (10) selon l'une quelconque des revendications 13 à 16, dans lequel les moyens de contrôle (54) peuvent être actionnés pour comparer les signaux de sortie de couleur pour identifier les zones correspondantes du tissu (18) par rapport aux signaux de sorties de position.

18. Appareil (10) selon l'une quelconque des revendications 13 à 17, dans lequel les moyens de contrôle (54) peuvent être actionnés pour analyser les signaux de sortie de couleur afin de calculer des valeurs de Delta E.

19. Procédé comprenant les étapes suivantes consistant à :
recevoir une séquence de signaux de sortie de couleur représentant la couleur de tissu d'une séquence de zones d'une longueur de tissu textile (18) ;
recevoir un signal de sortie de position respectif pour chacun des signaux de sortie de couleur ;
et déterminer la position de chacune des séquences de zones sur la longueur de tissu textile ;
fournir ainsi une séquence de signaux de sortie de couleur mesurés à partir des zones connues du tissu représentées par les signaux de sortie de position respectifs.

20. Procédé selon la revendication 19, dans lequel un dispositif d'alimentation (16) est utilisé pour produire un mouvement relatif entre un capteur (12) et la longueur de tissu textile (18) pour présenter une séquence de zones de tissu au niveau du capteur.

21. Procédé selon la revendication 20, dans lequel le mouvement relatif est produit par le déplacement de la longueur de tissu textile (18).

22. Procédé selon les revendications 20 ou 21, dans lequel la longueur de tissu textile (18) est déroulée à partir d'un rouleau (22), une zone déroulée étant présentée au niveau du capteur (12).

23. Procédé selon l'une quelconque des revendications 20 à 22, dans lequel la longueur de tissu textile (18) est déroulée à partir d'un premier rouleau (22) et enroulée sur un second rouleau (26), le tissu étant présenté au niveau du capteur (12) pendant qu'il est déroulé entre le premier rouleau et le second rouleau.

24. Procédé selon l'une quelconque des revendications 20 à 23, dans lequel la séquence de zones présentée au niveau du capteur (12) forme une ligne de positions (36) le long de la longueur de tissu textile (18).

25. Procédé selon l'une quelconque des revendications 20 à 24, dans lequel il existe une pluralité de capteurs (12).

26. Procédé selon la revendication 25, dans lequel la pluralité de capteurs (12) est disposée de manière à prendre les mesures à partir de lignes respectives (36) de zones de tissu, lorsque la longueur de tissu textile (18) se déplace par rapport aux capteurs.

27. Procédé selon les revendications 25 ou 26, dans lequel les champs de détection (14) de la pluralité de capteurs (12) sont disposés sensiblement perpendiculairement par rapport à la direction du mouvement relatif de la longueur de tissu textile (18) et des capteurs.

28. Procédé selon l'une quelconque des revendications 19 à 27, dans lequel les signaux de sortie de couleur et les signaux de sortie de position respectifs sont stockées.

29. Procédé selon l'une quelconque des revendications 19 à 28, dans lequel les signaux de sortie de couleur et les signaux de sortie de position respectifs sont assemblés pour former un ensemble de données représentant une carte (62) de la longueur de tissu textile (18).

30. Procédé selon la revendication 29, dans lequel un moyen d'affichage visuel (60) est utilisé pour fournir une représentation visuelle de la carte (62).

31. Procédé selon l'une quelconque des revendications 19 à 30, dans lequel les signaux de sortie de couleur sont analysés.

32. Procédé selon l'une quelconque des revendications 19 à 31, dans lequel les signaux de sortie de couleur de chaque longueur de tissu (18) sont analysés pour attribuer une mesure de couleur globale pour chaque longueur de tissu.

33. Procédé selon l'une quelconque des revendications 19 à 32, dans lequel les signaux de sortie de couleur sont analysés afin de déterminer la plage de couleurs représentée par les signaux de sortie de couleur pour une longueur de tissu (18).

34. Procédé selon la revendication 33, dans lequel le procédé détermine si la gamme de couleurs est supérieure à une gamme maximum autorisée.

35. Procédé selon l'une quelconque des revendications 19 à 34, dans lequel le procédé compare les signaux de sortie de couleur pour identifier les zones correspondantes du tissu (18) par rapport aux signaux de sorties de position.

36. Procédé selon l'une quelconque des revendications 19 à 35, dans lequel le procédé analyse les signaux de sortie de couleur pour calculer des valeurs de Delta E.

37. Logiciel qui, lorsqu'il est installé sur un système informatique (60), peut être actionné pour exécuter le procédé selon l'une quelconque des revendications 19 à 36.

38. Support lisible par ordinateur comportant des instructions lisibles par machine enregistrées dessus et représentant un logiciel selon la revendication 37.

39. Lot de coupons de tissu (72) découpés à partir de longueurs de tissu (18), les coupons de tissu étant identifiés en fonction des signaux de sortie de couleur et des signaux de sorties de position obtenus à l'aide de l'appareil (10) selon les revendications 1 à 18.

40. Lot de coupons de tissu (72) découpés à partir de longueurs de tissu (18), les coupons de tissu étant identifiés en fonction des signaux de sorties de couleur et des signaux de sorties de position obtenus selon le procédé selon revendications 19 à 36.
